## Europäisches Patentamt

## European Patent Office

## Office européen des brevets

(19)

(11) Veröffentlichungsnummer: **0 204 352**
**B1**

(12) **EUROPÄISCHE PATENTSCHRIFT**

(45) Veröffentlichungstag der Patentschrift:
**18.04.90**

(21) Anmeldenummer: 86200258.1

(22) Anmeldetag: **20.02.86**

(51) Int. Cl.⁴: **C07D 213/26,** C07D 215/12,
C07D 241/42

(54) Verfahren zur Chlorierung von Alkylseitenketten stickstoffhaltiger Heterocyclen.

(30) Priorität: **30.05.85  DE 3519364**

(43) Veröffentlichungstag der Anmeldung:
**10.12.86 Patentblatt 86/50**

(45) Bekanntmachung des Hinweises auf die Patenterteilung:
**18.04.90 Patentblatt 90/16**

(84) Benannte Vertragsstaaten:
**CH DE FR GB IT LI**

(56) Entgegenhaltungen:
ANGEWANDTE CHEMIE, Band 75, Nr. 5, 1963,
Seiten 235-240; W. MATHES et al.: "In der Seitenkette
halogenierte Methylpyridine und Methylchinoline"
THE JOURNAL OF ORGANIC CHEMISTRY, Band 35,
Nr. 3, März 1970, Seiten 719-722, The American
Chemical Society; E.C. JUENGE et al.: "Chlorination of
aromatic systems with trichloroisocyanuric acid under
polar and free-radical conditions"

(73) Patentinhaber: **RÜTGERSWERKE
AKTIENGESELLSCHAFT, Mainzer Landstrasse 217,
D-6000 Frankfurt am Main 1(DE)**

(72) Erfinder: **Jeromin, Günter Erich, Dr., Bergstrasse 14,
D-6900 Heidelberg(DE)**
Erfinder: **Orth, Winfried, Dr., Am Schachtelgraben 28,
D-6733 Hassloch/Pfalz(DE)**
Erfinder: **Fickert, Werner, Dr., Stockacher Strasse 14,
D-6800 Mannheim 61(DE)**

**Beschreibung**

Die Erfindung betrifft ein neues Verfahren zur Chlorierung von Alkylseitenketten stickstoffhaltiger Heterocyclen.

Es ist bekannt, daß z.B. die Chlorierung vcn Methylpyridinen mit Chlor schwierig ist und nur in geringer Ausbeute zu den Chlormethylpyridinen führt. Der bei der Reaktion entstehende Chlorwasserstoff führt zu einer Hemmung der Reaktion. Gemäß DBP 1 204 231 wurde diese Schwierigkeit dadurch umgangen, daß der Reaktionsmischung ein säurebindendes Mittel zugefügt wird, z.B. trockene Soda. Diesem Prinzip liegt auch das in JP-A 74 127 977 beschriebene Verfahren zugrunde. Nach FR-PS 1 394 362 wird die Seitenkettenchlorierung von Alkylpyridinen mit Chlor in Gegenwart von konzentrierter oder rauchender Schwefelsäure und einem Radikalerzeuger, z.B. Azo-isobuttersäurenitril oder UV-Licht beschrieben. Die extrem hohe Säurekonzentration soll eine Chlorierung des Kernes verhindern.

Die beschriebenen Verfahren weisen jedoch Nachteile auf. Alle Verfahren verwendeten das toxische und schwer zu handhabende und zu dosierende Chlor als Chlorierungsmittel und gemäß FR-PS 1 394 362 müssen bei der Aufarbeitung der Reaktionsprodukte erhebliche Mengen Schwefelsäure entsorgt werden. Außerdem benötigt dieses Verfahren in nicht geringer Menge das teure Azo-isobuttersäurenitril.

Die Erfindung hatte somit zur Aufgabe, ein Verfahren zur Chlorierung von Alkylseitenketten stickstoffhaltiger heterocyclischer Verbindungen bereitzustellen, bei dem ohne direkte Verwendung von Chlor und ohne Verwendung eines Radikalbildners die Chlorierung in einer gut zu dosierenden und einfach durchzuführenden Umsetzung erfolgt und bei der keine Entsorgungsprobleme bestehen.

Die Lösung der Aufgabe erfolgt durch ein Verfahren gemäß der Ansprüche.

Es wurde gefunden, daß sich Alkylgruppen stickstoffhaltiger Heterocyclen in einem üblichen und unter den gegebenen Reaktionsbedingungen inerten Lösemittel leicht und selektiv chlorieren lassen, wenn Trichlorisocyanursäure als Chlorierungsmittel verwendet wird.

Die Verwendung von Trichlorisocyanursäure als Chlorierungsmittel ist an sich bekannt. Gemäß J.Org.Chem. 31, 3836-3838 (1966) wird es zur Chlorierung cyclischer Ether oder gemäß Ann. 551,80 (1942) zur Chlorierung von Cyclohexen eingesetzt. Auch ist aus J.Org.Chem 35,719 (1970) bekannt, Trichlorisocyanursäure für die Chlorierung von aromatischen Kohlenwasserstoffen zu verwenden. Hierbei wird aber eindeutig unterschieden zwischen der säurekatalysierten Kernchlorierung und der, durch einen Radikalbildner, z.B. Benzoylperoxid katalysierten Seitenkettenchlorierung oder der Chlorierung von Naphthalin ohne Katalysator zu einem kernchlorierten Produkt.

Trotzdem ist es überraschend, daß die Chlorierung von alkylgruppenhaltigen N-Heterocyclen mit Trichlorisocyanursäure erfolgen kann, denn die Chlorierung mit dem bekannten und an sich üblichen N-Chlorsuccinimid ohne Katalysator verläuft bei dieser Verbindungsklasse negativ. Der Fachmann mußte also davon ausgehen, daß die Reaktion von Trichlorisocyanursäure mit z.B. 2-Methylpyridin ohne Radikalketteninitiator entweder zu keiner Reaktion oder zu einer Kernchlorierungsreaktion führt.

Es ist dabei ein weiterer Vorteil des erfindungsgemäßen Verfahrens, daß sich die alkylgruppenhaltigen N-Heterocyclen spontan und in guten Ausbeuten mit Trichlorisocyanursäure zu den chlorierten Verbindungen umsetzen. Dabei findet die Chlorierung in der Seitenkette statt. Kernchlorierte Produkte sind praktisch nicht nachzuweisen, obwohl diese zu erwarten waren, da die bei der Reaktion entstehende Cyanursäure durchaus eine Kernchlorierung katalysieren könnte.

Es findet zunächst eine Monochlorierung statt und im Überschuß des Chlorierungsmittels eine Polychlorierung der bereits chlorierten Alkylgruppe. Die Methode eignet sich für Alkylheterocyclen mit 1 bis 3 C-Atomen in der Alkylkette, die die Alkylgruppe in Nachbarstellung zum Heteroatom besitzen. Beispielsweise läßt sich α-Picolin leicht, γ-Picolin in geringerer Ausbeute und ß-Picolin nahezu nicht mehr chlorieren.

Das Verfahren zeichnet sich aus durch die Einfachheit der Durchführung, durch die geringe Toxizität, gute Handhabbarkeit und Dosierbarkeit des Chlorierungsmittels und seines Folgeproduktes. Insbesondere lassen sich säureempfindliche Verbindungen (z.B. Picoline) vorteilhaft chlorieren, ein Zusatz von Basen ist nicht nötig, da kein Chlorwasserstoff entsteht. Ebenso gestaltet sich die Aufarbeitung der Reaktionsmischung recht einfach. Die Reaktion kann vorteilhaft derart geführt werden, daß man den zu chlorierenden Alkylheterocyclus in einem geeigneten Lösemittel vorlegt und die Trichlorisocyanursäure bei der gewählten Temperatur portionsweise zugibt oder man legt das Chlorierungsmittel in dem Lösemittel vor und fügt den zu chlorierenden Alkylheterocyclus zu. Auch kann man bei kleineren Ansätzen, den Alkylheterocyclus, das Chlorierungsmittel und das Lösemittel bei Raumtemperatur vorlegen und langsam bis zum Anspringen der Reaktion hochheizen. Die Reaktion kann bei 20 - 200°C, vorzugsweise bei 40 - 100°C, durchgeführt werden. Niedrigere Reaktionstemperaturen verlangen längere Reaktionszeiten, höhere Temperaturen führen rascher zu höher chlorierten Produkten.

Um das Anspringen der Reaktion zu erleichtern, kann der Reaktionsmischung in geringer Menge ein Säureamid wie z.B. Dimethylformamid, Acetamid oder Benzamid zugefügt werden. Dieser Zusatz ist für die Durchführung der Chlorierung nicht unbedingt nötig, in vielen Fällen jedoch hilfreich und liefert unter Umständen bessere Ausbeuten.

Erfindungsgemäß zu chlorierende Verbindungen sind stickstoffhaltige Heterocyclen, die eine oder mehrere Alkylgruppen mit 1 bis 3 C-Atomen als Seitenketten besitzen, wobei die oder eine Seitenkette in

α-Stellung zu dem oder einem Stickstoffatom steht. Als stickstoffhaltige Heterocyclen können alle Grundkörper dienen, die ein quasiaromatisches Molekül haben, wie z.B. Pyridin, Pyridazin, Pyrimidin, Pyrazin, Chinolin, Triazin, Isochinolin, Chinoxalin oder Benzochinolin. Die alkylsubstituierten Stickstoffheterocyclen sind bei der Reaktion in einem Lösemittel gelöst, das unter den Bedingungen der Reaktion gegen Trichlorisocyanursäure inert ist. Beispiele für derartige Lösemittel sind: Methylenchlorid, Trichlormethan, Tetrachlormethan, Dichlorethan, Trichlorethan, Benzol, Chlorbenzol, Dimethylformamid, Alkane oder Cycloalkane.

Als Trichlorisocyanursäure kann die reine Verbindung, oder aber auch eine technische Qualität eingesetzt werden. Für die Chlorierungsreaktion stehen zwei Chloratome zur Verfügung, so daß man theoretisch auch Dichlorisocyansäure als Chlorierungsmittel einsetzen könnte. Die Menge an einzusetzender Trichlorisocyanursäure richtet sich nach dem gewünschten Chlorierungsgrad und es ist ein weiterer Vorteil des erfindungsgemäßen Verfahrens, daß bei Einsatz stöchiometrischer Mengen alkylsubstituierter N- Heterocyclen und Trichlorisocyanursäure nur eine Monochlorierung erfolgt. Erst bei weiterem Angebot größerer Mengen an Trichlorisocyanursäure werden die Monochlorpodukte zu Di- oder Trichlorprodukten chloriert.

Beispiele

Beispiel 1

Herstellung von 2-Chlormethylpyridin

200 g (2,15 Mol) α-Picolin und 7 g Benzamid werden in 750 ml Chloroform vorgelegt und die Lösung bis zum Rückfluß erhitzt. Jetzt fügt man portionsweise 270 g (1,16 Mol) Trichlorisocyanursäure (mind. 90 % verfügbares Chlor) innerhalb von 5 h zu. Zur Vervollständigung der Reaktion rührt man noch 5 h nach und läßt dann abkühlen. Um die ausgefallene Cyanursäure in Lösung zu bringen, versetzt man die Mischung unter Rühren mit 1200 ml Wasser und 400 g KOH, 50%-ig. Die Phasen werden getrennt, die wäßrige nochmals mit 250 ml Chloroform extrahiert und die vereinigten Chloroformphasen über MgSO$_4$ getrocknet. Das Chloroform wird vorsichtig bei 30°C abgedampft und der verbleibende Rückstand im Wasserstrahlvakuum destilliert. Nach einem geringen Vorlauf destillierten bei Kp$_{15}$ 77-82°C 181 g (66%) 2-Chlormethylpyridin, das sich rasch rot färbt.

Beispiel 2

Herstellung von 2-Chlormethylpyridin-hydrochlorid

200 g (2,15 Mol) 2-Methylpyridin und 14 g Dimethylformamid werden in 750 ml Chloroform gelöst, zum Rückfluß erhitzt und 300 g (1,29 Mol) Trichlorisocyanursäure (mind. 90 % verfügbares Chlor) ohne Heizung innerhalb von 50 min portionsweise zugegeben. Die Reaktion hält sich von selbst unter Rückfluß. Anschließend rührt man noch 2 h nach. Nach dem Abkühlen saugt man von der ausgefallenen Cyanursäure ab und wäscht die Chloroformlösung mit 100 ml NaOH, 5%-ig. Die Phasen werden getrennt, die Chloroformphase über MgSO$_4$ getrocknet und das Trockenmittel abfiltriert. Jetzt leitet man in die Chloroformlösung 100 g (2,74 mol) trockenen Chlorwasserstoff ein. Danach destilliert man das Chloroform im Vakuum ab und versetzt den Rückstand mit 250 ml trockenem Aceton. Man rührt die Mischung gründlich durch und saugt das ausgefallene Hydrochlorid ab. Es wird mit wenig Aceton nachgewaschen und getrocknet. Ausbeute: 185 g. Aus der Acetonmutterlauge kristallisieren beim Abkühlen im Kühlschrank nochmals 42 g. Ausbeute ingesamt: 227 g = 64,4 %. Fp. 120 - 122°C.

|  | gesamt Chlor | Chlorid |
|---|---|---|
| berechnet | 43,22 % | 21,61 % |
| gefunden | 43,92 % | 21,41 % |

Beispiel 3

Herstellung von 2-Chlormethyl-3-methylpyridin

107,16 g (1 Mol) 2,3-Dimethylpyridin und 5 g Benzamid werden in 400 ml Cyclohexan vorgelegt und zum Rückfluß erhitzt. Innerhalb von 4 h gibt man portionsweise 130 g (0,56 Mol) Trichlorisocyanursäure (mind. 90 % verfügbares Chlor) hinzu. Man rührt noch 7 h unter Rückfluß nach und läßt abkühlen. Unter Rühren versetzt man dann mit 500 ml Wasser und 150 ml KOH, 50 %-ig und bringt die ausgefallene Cyanursäure in Lösung. Die Phasen werden getrennt, die organische über MgSO$_4$ getrocknet und einge-

3

dampft. Der Rückstand wird im Vakuum destilliert. Nach einem Vorlauf von 0,5 g destillieren bei Kp03 53-57°C 85 g (60 %) 2-Chlormethyl-3-methylpyridin, das sich langsam rot färbt.

Beispiel 4

Herstellung von 2-Chlormethylchinolin

143,19 g (1 Mol) Chinaldin werden in 350 ml Chloroform gelöst, auf 60°C erhitzt und portionsweise innerhalb 175 min 83 g (0,36 Mol) Trichlorisocyanursäure (mind. 90 % verfügbares Chlor) zugegeben. Man rührt noch 45 min bei 60°C nach und läßt abkühlen. Um die ausgefallene Cyanursäure zu lösen, versetzt man mit 700 ml Wasser und 200 ml 50 %-igem KOH, rührt, bis zwei klare Schichten entstanden sind und trennt die Phasen. Die wäßrige Phase wird noch mit 2 x 150 ml Chloroform ausgerührt. Die vereinigten Chloroformphasen überschichtet man mit 350 ml 10%-iger HCl und rührt das 2-Monochlormethylchinolin aus der Chloroformlösung gründlich aus. Die Schichten werden getrennt, die Chloroformphase über MgSO4 getrocknet und am Rotationsdampfer eingedampft. Es bleiben 52 g (24,5 %) rohes Dichlormethylchinolin übrig. Nach Umkristallisation aus Isopropanol verbleiben 16 g (7,5 %), Schmelzpunkt 80 - 81 °C, Chlorgehalt: 33,02 %, Theorie 33,4 %. Die saure wäßrige Phase wird mit 300 ml Essigester überschichtet, mit NaOH, 50%-ig auf pH 3 gebracht und die Phasen gut durchmischt. Anschließend trennt man die Phasen, rührt die wäßrige Phase nochmals mit 100 ml Essigester aus, vereinigt die Essigesterphasen und trocknet über MgSO4. Nach dem Abdestillieren hinterbleiben 95 g (53,5 %) kristallines Chlormethylchinolin, Fp. 53°C, Chlorgehalt: 19,87 %, Theorie: 19,96 %. Die wäßrige Phase wird mit Natronlauge alkalisch gemacht und mit 200 ml Essigester extrahiert. Nach dem Trocknen und Abdampfen des Essigesters erhält man 23 g Chinaldin zurück. Ausbeute an 2-Chlormethylchinolin, berechnet auf umgesetztes Chinaldin: 63,7 %.

Beispiel 5

Herstellung von 2-(1-Chlorethyl)-pyridin

107,16 g (1 Mol) 2-Ethylpyridin werden in 400 ml Ethylenchlorid gelöst, die Lösung zum Rückfluß erhitzt und innerhalb 5 h 150 g (0,65 Mol) Trichlorisocyanursäure (mind. 90 % verfügbares Chlor) portionsweise zugegeben. Man rührt noch 2 h nach, läßt abkühlen und saugt die ausgefallene Cyanursäure ab. Die Chloroformlösung wird mit 50 ml KOH, 5 %-ig, gewaschen, über MgSO4 getrocknet und eingedampft. Der Rückstand wird über eine Kolonne destilliert. Kp0,5 45 - 50°C, 53,5 g Rohprodukt. Zur Reinigung löst man das Rohprodukt in 150 ml Chloroform und extrahiert mit 150 g HCl, 10%-ig. Die saure Phase wird dann mit NaOH auf pH 4 abgestumpft und mit 150 ml Essigester extrahiert. Nach dem Trocknen und Eindampfen bleiben 35,5 g (25,1 %) 2-(1-Chlorethyl)pyridin. Chlorgehalt: 25,73 %, Theorie: 25,04 %.

Beispiel 6

Herstellung von 2-Chlormethyl-chinoxalin

72,1 g (0,5 Mol) 2-Methylchinoxalin werden in 250 ml Chloroform gelöst und auf Rückfluß erhitzt. Jetzt gibt man unter Rühren innerhalb von 1,5 h 52 g (0,22 Mol) Trichlorisocyanursäure (mind. 90 % verfügbares Chlor) portionsweise hinzu und rührt noch 30 min nach. Nach dem Abkühlen versetzt man mit 350 ml Eiswasser und alkalisiert unter Kühlung bei 5 - 10°C mit 65 ml KOH, 50%-ig. Die Phasen werden anschließend getrennt, die Wasserphase nochmals mit 100 ml Chloroform extrahiert und die vereinigten Chloroformphasen getrocknet und eingedampft. Rückstand: 88 g gelbe Kristalle. 88 g Rohprodukt werden aus 70 ml n-Hexan umkristallisiert. Ausbeute: 67 g (75 %), Fp: 45 - 46°C (Zersetzung).

Analyse:

|  | C | H | N | Cl |
|---|---|---|---|---|
| berechnet | 60,52 | 3,95 | 15,68 | 19,85 % |
| gefunden | 59,9 | 3,8 | 15,5 | 19,97 % |

Beispiel 7

Herstellung von 2-Chlormethyl-4,6-dimethylpyridin

121,18 g (1 Mol) 2,4,6-Collidin werden in 400 ml Chloroform gelöst, zum Rückfluß erhitzt und portionsweise innerhalb von 4 h 140 g (0,6 Mol) Trichlorisocyanursäure (mind. 90 % verfügbares Chlor) eingetragen. Es werden noch 30 min nachgerührt und nach dem Abkühlen die ausgefallene Cyanursäure abfiltriert. Die Chloroformlösung wird nun mit 150 ml HCl, 10%-ig, versetzt und das Produkt in die wäßrige Phase überführt. Die saure, wäßrige Phase wird nach dem Abtrennen mit 150 ml Essigester versetzt und mit NaOH alkalisch gemacht.
Die Phasen werden getrennt, die wäßrige Phase nochmals mit 100 ml Essigester ausgeschüttelt und die vereinigten Essigesterphasen getrocknet und eingedampft. Der Rückstand wird an der Oelpumpe destilliert. Bei Kp₁ 64 - 70°C destillieren 45 g (28,9 %) 2-Chlormethyl-4,6- dimethylpyridin. Cl-Gehalt gefunden: 22,78 % Cl-Gehalt Theorie: 22,78 %.

Vergleichsbeispiel

Versuch der Chlorierung mit N-Chlorsuccinimid

200 g (2,15 Mol) 2-Methylpyridin werden in 750 ml Chloroform gelöst, auf Rückfluß erhitzt und portionsweise innerhalb von 5 h 287,1 g (2,15 Mol) N-Chlorsuccinimid (98 % aktives Chlor) zugegeben. Nach beendeter Zugabe wurde noch 5 h unter Rückfluß erhitzt. Es konnte dünnschichtchromatographisch kein nennenswerter Umsatz beobachtet werden.

**Patentansprüche**

1. Verfahren zur Chlorierung von Alkylseitenketten mit 1 bis 3 C-Atomen stickstoffhaltiger Heterocyclen, die in alpha-Stellung zu dem oder einem Stickstoffatom stehen, dadurch gekennzeichnet, daß diese Heterocyclen bei Temperaturen im Bereich von 20 bis 200°C mit Trichlorisocyanursäure zur Reaktion gebracht werden.
2. Verfahren nach Anspruch 1, dadurch gekennzeichnet, daß die Reaktion im Temperaturbereich von 40 bis 100°C durchgeführt wird.
3. Verfahren nach Anspruch 1 oder 2, dadurch gekennzeichnet, daß vor Beginn der Reaktion 1 bis 10 Gew.-%, bezogen auf die Menge an stickstoffhaltigen Heterocyclen, eines Carbonsäureamids zugegeben werden.

**Claims**

1. A process for the chlorination of alkyl side chains with 1 to 3 carbon atoms of nitrogen-containing heterocycles, which are in the alpha position with respect to the or a nitrogen atom, characterized in that the said heterocycles are reacted with trichloroisocyanuric acid at temperatures in the range of from 20 to 200°C.
2. A process according to Claim 1, characterized in that the reaction is carried out in the temperature range of from 40 to 100°C.
3. A process according to Claim 1 or 2, characterized in that before the beginning of the reaction from 1 to 10% by weight, relative to the quantity of nitrogen-containing heterocycles, of a carboxylic acid amide is added.

**Revendications**

1. Procédé de chloruration de chaînes alkyles latérales avec 1 à 3 atomes de C d'hétérocycles contenant de l'azote, qui se trouvent en position alpha par rapport à l'atome ou à un atome d'azote, caractérisé en ce que lesdits hétérocycles sont mis à réagir avec de l'acide trichloro-isocyanurique à des températures dans le domaine de 20 à 200°C.
2. Procédé de la revendication 1, caractérisé en ce que la réaction est effectuée dans un domaine de température de 40 à 100°C.
3. Procédé selon la revendication 1 ou 2, caractérisé en ce qu'avant le début de la réaction, on ajoute 1 à 10% en poids, par rapport à la quantité d'hétérocycles contenant de l'azote, d'un amide d'acide carboxylique.